# EUROPÄISCHE PATENTSCHRIFT

(11) **EP 1 230 831 B1**
(45) Veröffentlichungstag und Bekanntmachung des Hinweises auf die Patenterteilung: **25.06.2003**
(21) Anmeldenummer: 00972818.9
(22) Anmeldetag: 20.10.2000
(51) Int. Cl.: H05K 9/00, A61N 1/16

(54) **VORRICHTUNG UND VERFAHREN ZUR MINIMIERUNG ELEKTROMAGNETISCHER EMISSIONEN TECHNISCHER EMITTENTEN**
DEVICE AND METHOD FOR MINIMISING ELECTROMAGNETIC EMISSIONS OF TECHNICAL EMITTERS
DISPOSITIF ET PROCEDE PERMETTANT DE MINIMISER LES EMISSIONS ELECTROMAGNETIQUES ISSUES D'EMETTEURS TECHNIQUES

(30) Priorität: 19.11.1999 DE 19955974; 10.02.2000 DE 10005905
(43) Veröffentlichungstag der Anmeldung: 14.08.2002
(73) Patentinhaber: Reichwein, Dietrich, 5700 Zell am See (AT)
(72) Erfinder: Reichwein, Dietrich, 5700 Zell am See (AT)
(74) Vertreter: Pfenning, Meinig & Partner GbR
(86) Internationale Anmeldenummer: EP0010325
(87) Internationale Veröffentlichungsnummer: WO01039567

(56) Entgegenhaltungen:
- EP-A- 0 880 311
- WO-A-00/74461
- DE-A- 3 938 238
- DE-A- 19 850 238
- US-A- 685 957

## Beschreibung

Die vorliegende Erfindung bezieht sich auf eine Vorrichtung und ein Verfahren zur Minimierung elektromagnetischer Emissionen technischer Emittenten, d.h. zur Minimierung von Potentialwirbelanteilen elektromagnetischer Wechselfelder aus technischen Geräten. Der Stand der Wissenschaft belegt in umfangreichen Untersuchungen, daß nicht - wie vermutet - das magnetische Wechselfeld allein durch Induktion, sondern vielmehr mittels unterschiedlicher Formation und Dichte von Ring- bzw. Potentialwirbelsystemen agiert (K. Meyl "Elektromagnetische Umweltverträglichkeit, Ursachen, Phänomene und naturwissenschaftliche Konsequenzen. Umdruck zur Vorlesung", ISBN 3-9802 642-8-3 und ISBN 3-9802-542-9-1. sowie K. Meyl "Potentialwirbel" Bd. 1 und Bd. 2, ISBN 3-9802-542-1-6 und ISBN 3-9802-542-2-4).

Da diese Potentialwirbel (elektromagnetische Longitudinalwellenformen) nahezu verlustlos alle Arten von Abschirmungen durchdringen, ist die Wirkungslosigkeit von Abschirmvorrichtungen aller Art, wie sie aus dem Stand der Technik bekannt sind, weiter nicht überraschend. In der Energietechnik wurde allerdings die Verwendung von Longitudinalwellen, mit denen sich Potentialwellen fortpflanzen, um die Jahrhundertwende zum 20. Jahrhundert eingestellt. Dennoch seien hier die US-Patente 513.138 (1897), 645,576 (1900) sowie 685 957 (1901) von Nikola TESLA angeführt.

Zu Potentialwirbelablösungen aus dem Lateralwellenfeld (Transversalwellenfeld) kommt es bei freien unter Spannung stehenden Leitungsenden (offene Schalter), bei abruptem Spannungs- oder Stromanstieg (z.B. Wechselrichter) sowie bei disruptiven Entladungen sowie bei Kathodenstrahlröhren, beispielsweise in Bildschirmen, sowie nahezu bei allen digitalen Datenübertragungen, ausgenommen Lichtübertragungen in Lichtleitern wie Glasfasern mit Totalreflexion.

Die DE 198 50 238 A1 offenbar eine Vorrichtung zur Minimierung elektromagnetischer Emissionen technischer Emittenten, bei der mittels einer geschlossenen Induktionsschlaufe in Form einer Möbiuswindung Potentialwirbel absorbiert, respektive in Lateralwellen umgeformt werden. Diese Vorrichtung ist jedoch auf den durch die Eigeninduktivität der Möbiuswindung gegebenen Frequenzbereich eingegrenzt.

Aufgabe der vorliegenden Erfindung ist es daher, eine Vorrichtung und ein Verfahren zur Verfügung zu stellen, mit denen eine wirkungsvolle Minimierung elektromagnetischer Emissionen technischer Emittenten erzielt werden kann.

Diese Aufgabe wird durch die Vorrichtung nach Anspruch 1 und das Verfahren nach Anspruch 10 gelöst. Vorteilhafte Weiterbildungen der erfindungsgemäßen Vorrichtung werden in den abhängigen Ansprüchen gegeben.

Das erfindungsgemäße Verfahren und die erfindungsgemäße Vorrichtung unterscheiden sich vom Verfahren nach der DE 198 50 238 A1 dadurch, daß es frequenzunabhängig das Bestreben freier Wirbel, sich um einen responsfähigen Einleiter offener Art (in Anlehnung der vorstehend beschriebenen US-Patente) zu wickeln, bedient, indem weiterhin vorteilhafterweise die Tatsache genutzt wird, daß Potentialwirbel beispielsweise in der Verarmungszone eines p-n-Überganges zu Lateralwellenformen kollabieren und nunmehr über bekannte. Zweileitersysteme im geschlossenen Leiter als Gleichstrom, beispielsweise über Widerstände in Wärmeenergie, abgeleitet werden können.

Das erfindungsgemäße Verfahren und die erfindungsgemäße Vorrichtung nutzen also die Tatsache, daß Potentialwirbel technischer Genese die Eigenschaft haben, sich um Fäden mit Feldrespons zu zentrieren. Dies können dünne Drähte mit Ruhestrom oder solche aus ferromagnetischem Material, wie es auch in den oben genannten US-Patenten erwähnt ist, sein. Solcher Art ist auch der Zusammenhang der Wechselwirkungen potentialwirbelablösender technischer Einrichtung definiert. Sinusförmiger Wechselstrom im Lateralwellenbereich ("Maxwell"-sche Gleichungen), wie er von den Energieversorgungsunternehmen beispielsweise in Haushalte geliefert wird, ist nur der eine technisch meßbare Anteil. Der andere Anteil geht immer von dem Longitudinalwellenanteil aus, und zwar in Form von Ablösungen von Potentialwirbeln. Dies ist immer und ausnahmslos beim Verbraucher zu beobachten. Über konstruktive Maßnahmen hinaus, um besagte Potentialwirbelablösung von vornherein zu unterbinden, ist durch das erfindungsgemäße Verfahren und die erfindungsgemäße Vorrichtung eine weitere Minimierung bereits abgelöster Potentialwirbel und deren Abgabe an die Umwelt erzielbar.

Insgesamt ergibt sich folglich erfindungsgemäß eine Vorrichtung, bei der ein Einleiter, der an dem Emittenten anordenbar ist, mit einer Vorrichtung zur Umwandlung von elektromagnetischen Longitudinalwellen in elektromagnetische Lateralwellen, vorteilhafterweise mit einem p-n-Übergang auf Seiten des p-Bereiches, verbunden ist. Der n-Bereich des p-n-Überganges ist mit einer Vorrichtung zur Umwandlung von elektromagnetischen Lateralwellen elektrisch leitend verbunden. Dies kann beispielsweise ein Widerstand sein, durch den die Lateralwellen in bekannter Weise gemäß den Maxwell'schen Gleichungen in Wärme umgewandelt werden.

Vorteilhafterweise wird der p-n-Übergang als Zenerdiode realisiert. Der Einleiter wird vorteilhafterweise mit dem Gehäuse des Emittenten verbunden, sofern dieses aus Metall ist bzw. sofern dieses mit der Erde verbunden ist. Der Einleiter kann selbst auch geerdet werden. Zur Vermeidung von Eigeninduktivitäten in an sich bekannter Art wird der Emittent vorteilhafterweise schlaufenartig in bifilaren Windungsformen von dem Einleiter umschlungen.

Im Folgenden werden einige Beispiele der erfindungsgemäßen Vorrichtung und des erfindungsgemäßen Verfahrens beschrieben.

Es zeigen:
- Fig. 1: eine erfindungsgemäße Vorrichtung;
- Fig. 2: eine weitere erfindungsgemäße Vorrichtung;
- Fig. 3: eine erfindungsgemäße Vorrichtung;
- Fig. 4: eine erfindungsgemäße Vorrichtung, und
- Fig. 5: eine erfindungsgemäße Vorrichtung.

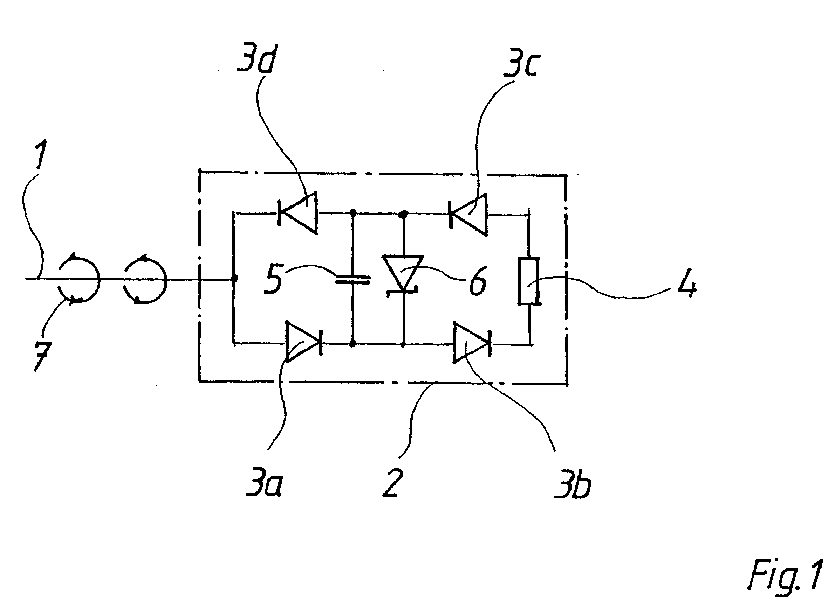

Fig. 1 zeigt beispielhaft die Grundanordnung einer erfindungsgemäßen Vorrichtung in einer ihrer Ausgestaltungsformen.

Ein Ringwirbelabsorber 1 (responsfähiger Faden/Einleiter) leitet Longitudinalwellen (Ringwirbel 7) zu einem seriell angeordneten Diodenpaar aus zwei Dioden 3a und 3b. An diesem Diodenpaar kollabieren die Potentialwirbel. Die ankommende Longitudinalwellenfrequenz kann über Dioden mittels eines Oszilloskops gemessen werden. Es stellt sich dabei heraus, daß diese Frequenz vorwiegend Netzfrequenz enthält, auf der höhere Frequenzen aufgeprägt sind ("reiten"). Der Energieinhalt der kollabierten Potentialwirbel findet sich im Kondensator 5 nach W = U² .C/2 bzw. am Widerstand 4 nach P = I² .R wieder. Damit kann auch die Wirksamkeit der Vorrichtung bewertet werden.

Die Dioden 3a und 3b sind in Reihe geschaltet mit dem Widerstand 4, an den sich weitere zwei Dioden 3c und 3d anschließen, die in einem Ringschluß wieder mit dem Ringwirbelabsorber 1 verbunden sind. Zwischen der Leitung zwischen den Dioden 3a und 3b sowie der Leitung zwischen den Dioden 3c und 3d ist zum einen der genannte Kondensator 5 angeordnet, sowie parallel zu diesem zwischen diesen beiden Leitungen ein Überspannungsschutz 6, beispielsweise eine Zenerdiode oder ein Varistor. Die Gesamtanordnung bildet einen Lateralwellenwandler 2, wobei die von dem Einleiter 1 aufgefangenen Ringwirbel 7 in der Verarmungszone der p-n-Übergänge der Dioden in Lateralwellen umgewandelt werden.

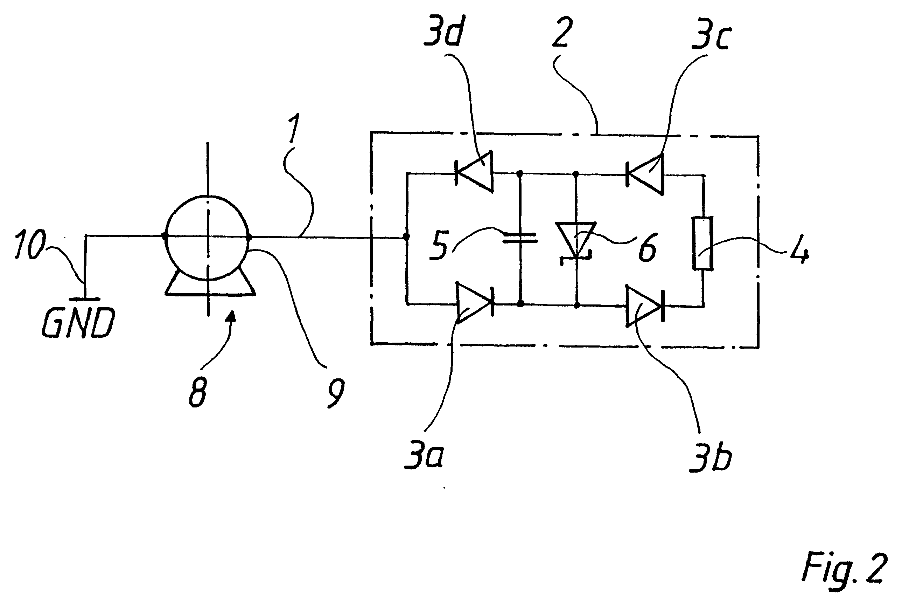

Fig. 2 zeigt eine weitere erfindungsgemäße Vorrichtung, wobei hier wie auch bei den folgenden Figuren gleiche Bezugszeichen gleiche Elemente bezeichnen, so daß deren Beschreibung fortgelassen wird.

Wie in Fig. 2 dargestellt, erhöht sich die Wirksamkeit der erfindungsgemäßen Absorbervorrichtung, wenn noch vor der Ringwirbelablösung das Longitudinalwellenfeld am vorzugsweise auf Erdpotential bezogenen Gehäuse 9 eines Emittenten Teil des Einleiters ist. Das Erdpotential ist in Fig. 2 und in den folgenden Figuren mit GND und mit Bezugszeichen 10 versehen.

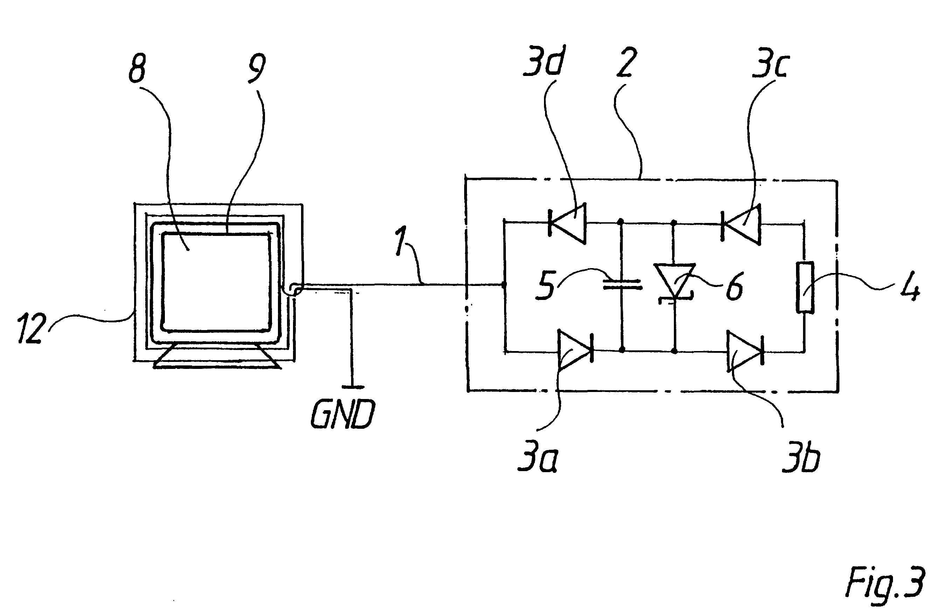

Fig. 3 zeigt eine weitere Vorrichtung für den Fall, daß ein Gehäuse 9 eines Emittenten 8 aus nicht leitfähigem Material hergestellt ist. In diesem Fall wird der absorbierende Einleiter 1 als Bifilarschlaufe 12 um das Gehäuse 9 gewunden. Dadurch, daß der Einleiter 1 als Bifilarschlaufe um das Gehäuse gewunden wird, wird eine irequenzeinschränkende Selbstinduktion vermieden und der Einleiter 1 nimmt daher jede Frequenz an, da die Bifilarwicklung induktivitätsunabhängig ist.

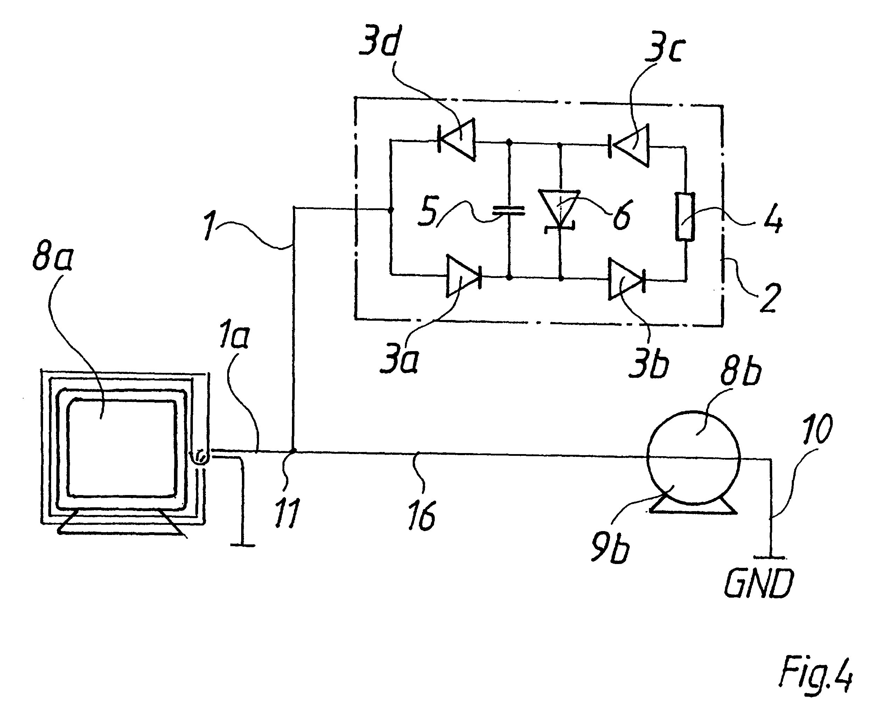

Fig. 4 zeigt eine weitere erfindungsgemäße Vorrichtung, an der zwei unterschiedliche Emittenten 8a und 8b angeschlossen sind. Denn ein erfindungsgemäßer Absorber kann mehrere Emittenten sowohl seriell als auch parallel abschirmen. In dem in Fig. 4 dargestellten Falle, bei dem ein Gerät 8a mittels bifilarer Wicklung mit dem Einleiter 1 verbunden ist und das Gehäuse 9b eines Gerätes 8b sowohl mit der Erde 10 als auch mit dem Einleiter über die Leitung 1b verbunden ist, wobei die beiden Einleiter 1a und 1b von den Geräten 8a und 8b an einem Knotenpunkt 11 mit dem Stammeinleiter 1 verbunden sind, zerfallen Ringwirbel teilweise von den Einleitern 1a und 1b, wenn sie sich im Knotenpunkt 11 treffen. Es ist dabei zu betonen, daß der Einleiter 1 nicht zwingend geerdet sein muß.

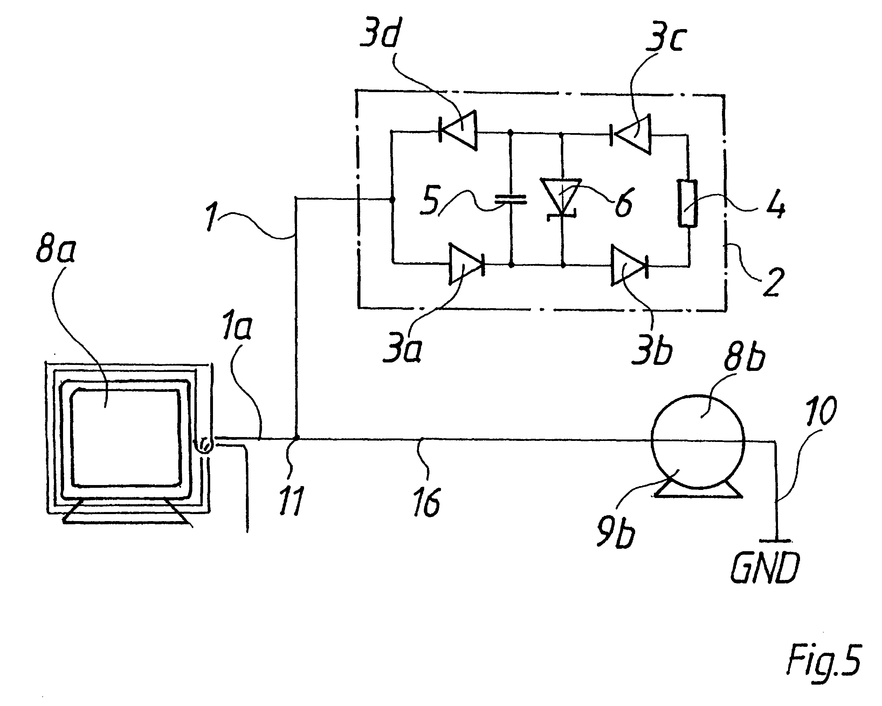

Fig. 5 zeigt ein weiteres Beispiel, das dem in Fig. 4 dargestellten Beispiel entspricht, wobei hier lediglich der Einleiter 1b über das Gehäuse 9b des Gerätes 8b mit einer Erdung 10 verbunden ist.

Zusammenfassend läßt sich feststellen, daß das Verfahren sich einer Vorrichtung zum Absorbieren abgelöster Ringwirbel bzw. Longitudinalwellen und eines Lateralwellenwandlers bedient, die, wie vorstehend dargestellt, ausgeführt werden, wobei relevante Longtitudinalwellen über Einleitertechnik einem Lateralwellenwandler zugeführt werden, in dem in p-n-Uberqängen von Halbleitern, vorzugsweise Dioden, die longitudinal fortschreitenden Potentialwirbel in Gleichströme umgesetzt und beispielsweise über Widerstände in Wärmeenergie umgewandelt werden.

## Patentansprüche

1. Vorrichtung zur Minimierung elektromagnetischer Emissionen technischer Emittenten,
**gekennzeichnet durch**
einen an dem Emittenten anordenbaren elektromagnetische Longitudinalwellen absorbierenden elektrischen Leiter,
eine Vorrichtung zur Umwandlung von elektromagnetischen Longitudinalwellen in elektromagnetische Lateralwellen sowie
eine Vorrichtung zur Umwandlung von elektromagnetischen Lateralwellen, wobei der Leiter über die Vorrichtung zur Umwandlung von elektromagnetischen Longitudinalwellen mit der Vorrichtung zur Umwandlung von elektromagnetischen Lateralwellen elektrisch leitend verbunden ist.

2. Vorrichtung nach dem vorhergehenden Anspruch, **dadurch gekennzeichnet, daß** die Vorrichtung zur Umwandlung von elektromagnetischen Longitudinalwellen einen p-n-Übergang aufweist, der auf Seiten des p-Bereiches mit dem Einleiter und auf Seiten des n-Bereiches mit der Vorrichtung zur Umwandlung von elektromagnetischen Lateralwellen elektrisch leitend verbunden ist.

3. Vorrichtung nach dem vorhergehenden Anspruch, **dadurch gekennzeichnet, daß** der p-n-Übergang eine Zenerdiode ist.

4. Vorrichtung nach einem der vorhergehenden Ansprüche, **dadurch gekennzeichnet, daß** die Vorrichtung zur Umwandlung von elektromagnetischen Lateralwellen ein Widerstand ist.

5. Vorrichtung nach einem der vorhergehenden Ansprüche, **dadurch gekennzeichnet, daß** der Einleiter mit dem Gehäuse des Emittenten verbindbar ist.

6. Vorrichtung nach einem der vorhergehenden Ansprüche, **dadurch gekennzeichnet, daß** der Einleiter geerdet ist.

7. Vorrichtung nach einem der vorhergehenden Ansprüche, **dadurch gekennzeichnet, daß** der Einleiter bifilar um den Emittenten wickelbar ist.

8. Vorrichtung nach einem der vorhergehenden Ansprüche, **dadurch gekennzeichnet, daß** der Einleiter an mehreren Emittenten in serieller Weise anordenbar ist.

9. Vorrichtung nach einem der vorhergehenden Ansprüche, **dadurch gekennzeichnet, daß** der Einleiter an einer Verbindungsstelle mit einer kreisförmig seriellen elektrischen Verschaltung von vier Dioden verbunden ist, wobei zwischen der von der Verbindungsstelle her gezählten zweiten und dritten Diode ein Widerstand angeordnet ist und zwischen der Verbindungsleitung von der ersten zur zweiten Diode und der Verbindungsleitung von der dritten zur vierten Diode eine Kapazität und eine Überspannungsschutzvorrichtung parallel zueinander angeordnet sind.

10. Vorrichtung nach einem der vorhergehenden Ansprüche, **dadurch gekennzeichnet, daß** die Überspannungsschutzvorrichtung ein Varistor oder eine Zenerdiode ist.

11. Verfahren zur Minimierung elektromagnetischer Emissionen eines technischen Emittenten, **dadurch gekennzeichnet, daß** an dem Emittenten eine Vorrichtung nach einem der vorhergehenden Ansprüche angeordnet wird.

## Claims

1. Device for minimising electro-magnetic emissions of technical emitters, **characterised by**
an electric conductor which is arranged on an emitter and which absorbs electro-magnetic longitudinal waves,
a device for conversion of electro-magnetic longitudinal waves into electro-magnetic lateral waves, as well as
a device for conversion of electro-magnetic lateral waves, and the conductor is via the device for conversion of electro-magnetic longitudinal waves in an electrically conducting manner connected to the device for conversion of electro-magnetic lateral waves.

2. Device according to the above claim, **characterised in that** the device for conversion of electro-magnetic longitudinal waves comprises a p-n-transition which is at the side of a p-range in an electrically conducting manner connected to the feeder, and at the side of the n-range to the device for conversion of electro-magnetic lateral waves.

3. Device according to the above claim, **characterised in that** the p-n-transition is a Zener diode.

4. Device according to one of the above claims, **characterised in that** the device for conversion of electro-magnetic lateral waves is a resistor.

5. Device according to one of the above claims, **characterised in that** the feeder is connectable to the casing of the emitter.

6. Device according to one of the above claims, **characterised in that** the feeder is earthed.

7. Device according to one of the above claims, **characterised in that** the feeder is in a bifilar manner wound around the emitter.

8. Device according to one of the above claims, **characterised in that** the feeder can be arranged in series on a plurality of emitters.

9. Device according to one of the above claims, **characterised in that** the feeder is at a connecting point connected to a circular series electrical circuit of four diodes, and between the second and third diode, as counted from the connecting point, is placed a resistor, and between the connecting line from the first diode to the second and the connecting line from the third to the fourth diode are arranged in parallel a capacitor and a surge protector.

10. Device according to one of the above claims, **characterised in that** the surge protector is a varistor or a Zener diode.

11. Method for minimising electro-magnetic emissions of a technical emitter, **characterised in that** the emitter is fitted with a device according to one of the above claims.

## Revendications

1. Dispositif pour minimiser des émissions électromagnétiques d'émetteurs techniques, **caractérisé par** un conducteur électrique qui peut être disposé sur l'émetteur et absorbe des ondes électromagnétiques longitudinales; un dispositif pour convertir des ondes électromagnétiques longitudinales en des ondes électromagnétique latérales, ainsi qu'un dispositif pour convertir des ondes électromagnétiques latérales, le conducteur étant relié d'une manière électriquement conductrice au moyen du dispositif servant à convertir des ondes électromagnétiques longitudinales, au dispositif servant à convertir des ondes électromagnétiques latérales.

2. Dispositif selon la revendication précédente, **caractérisé en ce que** le dispositif servant à convertir des ondes électromagnétiques longitudinales comporte une jonction p-n, qui est relié d'une manière électriquement conductrice, du côté de la zone p au conducteur unifilaire et du côté de la zone n, au dispositif pour réaliser la conversion d'ondes électromagnétiques latérales.

3. Dispositif selon la revendication précédente, **caractérisé en ce que** la jonction p-n est une diode Zener.

4. Dispositif selon l'une des revendications précédentes, **caractérisé en ce que** le dispositif de conversion d'ondes électromagnétiques latérales est une résistance.

5. Dispositif selon l'une des revendications précédentes, **caractérisé en ce que** le conducteur unifilaire peut être relié au boîtier de l'émetteur.

6. Dispositif selon l'une des revendications précédentes, **caractérisé en ce que** le conducteur unifilaire est connecté à la terre.

7. Dispositif selon l'une des revendications précédentes, **caractérisé en ce que** le conducteur unifilaire peut être enroulé d'une manière bifilaire autour de l'émetteur.

8. Dispositif selon l'une des revendications précédentes, **caractérisé en ce que** le conducteur unifilaire peut être disposé en série sur plusieurs émetteurs.

9. Dispositif selon l'une des revendications précédentes, **caractérisé en ce que** le conducteur unifilaire est relié à un point de jonction avec un circuit électrique série de forme circulaire formé de quatre diodes, une résistance étant disposée entre les seconde et troisième diodes, comptées à partir du point de jonction, et une capacité et un dispositif de protection contre les surtensions étant disposés en parallèle, entre la ligne de jonction s'étendant de la première diode à la seconde diode et la ligne de jonction s'étendant de la troisième à la quatrième diode.

10. Dispositif selon l'une des revendications précédentes, **caractérisé en ce que** le dispositif de protection contre les surtensions est une varistance ou une diode Zener.

11. Procédé pour minimiser des émissions électromagnétiques d'un émetteur technique, **caractérisé en ce qu'**un dispositif selon l'une des revendications précédentes est disposé sur l'émetteur.
